# EUROPEAN PATENT APPLICATION

(11) **EP 4 759 793 A1**
(43) Date of publication of application: **17.06.2026**
(21) Application number: 24220135.8
(22) Date of filing: 16.12.2024
(51) Int. Cl.: C07C 45/75, C07C 45/81, C07C 45/82, C07C 47/22

(54) **OPTIMIZED PROCESSING OF REACTOR EFFLUENT IN THE SYNTHESIS OF METHACROLEIN FROM PROPIONALDEHYDE AND FORMALDEHYDE**

(71) Applicant: Röhm GmbH, 64295 Darmstadt (DE)
(72) Inventor: The designation of the inventor has not yet been filed
(74) Representative: Röhm Patent Association

(57) **Abstract**

The present invention relates to a process for preparing methacrolein starting from formaldehyde and propionaldehyde in the presence of a homogenous catalyst mixture. The obtained reaction mixture is partially transferred into a first distillation column which comprises at least one column internal. The reaction mixture is introduced into the first distillation column partially above the at least one column internal and partially below the at least column internal.

## Description

### Field of the invention

The present invention relates to a process for preparing methacrolein starting from formaldehyde and propionaldehyde in the presence of a homogenous catalyst mixture. The obtained reaction mixture is partially transferred into a first distillation column which comprises at least one column internal. The reaction mixture is introduced into the first distillation column partially above the at least one column internal and partially below the at least column internal.

### Prior Art

WO 2016/042000 A1 discloses a method for producing methacrolein starting from formaldehyde and propionaldehyde in the presence of a catalyst mixture comprising dimethyl amine and acetic acid. The reaction is carried out in a tubular or a plate reactor with a feed temperature in the range from 100 °C to 150 °C and an outlet temperature of not greater than 180 °C. The water content in the feed of the reactor is in the range from 45 wt.-% to 85 wt.-%. The amount of organic base in the reactor feed is more than 5 mol-% based on propionaldehyde. The pressure in the reactor is higher than the boiling pressure of the reaction mixture and the residence time of the reaction mixture in the reactor is between 1 s and 30 s. The obtained reaction mixture is discharged into a column and stripped with water steam. The product is obtained at the top of the column, condensed and separated into an upper and a lower phase in a phase separator. The upper phase comprises methacrolein, the lower phase comprises essentially water. The lower phase may be recycled into the column. No further treatment of the recycled lower phase is disclosed in WO 2016/042000 A1.

EP 3 786 146 A1 discloses a method for treating the reactor outlet of a methacrolein synthesis. The reactor outlet is expanded in an expansion vessel to obtain a gaseous and a liquid phase. The gaseous phase is condensed and introduced into a phase separator, in which a methacrolein-containing phase and a water-containing phase are obtained. The liquid phase of the expansion vessel is transferred into a column to extract methacrolein. The overhead stream of the column is also condensed and introduced into the phase separator. The bottom stream of the column is partially recycled into the reactor and partially discharged as waste.

A process for the preparation of methacrolein (MAL) is described in DE 3 213 681 which is particularly characterized in that the reaction is carried out at a temperature of greater than 150 °C with a reaction time of at most 25 min in the presence of secondary amines and optionally of acids. In the best case, propionaldehyde is reacted with formalin at temperatures of 162 °C to 205 °C and a residence time of 6 seconds. The yield in the best case is 97.2% and the DIMAL content is low, but almost 1% by weight. The water content in the feed is 40% by weight and the amine concentration is 2.5% by weight based on the water content. The distinctly low yield and the comparatively high DIMAL content show that this method is less advantageous. The catalyst solution is recycled after distilling off water and methacrolein.

US 4,408,079 describes a process for preparing MAL in which the reaction of propionaldehyde with formalin is carried out at a molar ratio of between 0.9 and 1.5 to 1, a pH between 2.5 and 7 and temperatures of 0 °C to 150 °C in the presence of a secondary amine at a concentration of 0.025 to 0.75 or of 0.05 to 1.5 mol, and organic acids at a concentration of 0.05 to 1.5 mol, based in each case on 1 mol of propionaldehyde. In comparison to the teaching of DE 3 213 681, the selected temperature range is therefore significantly lower. According to US 4,408,079, the reaction is carried out continuously in a stirred tank cascade of two to three reactors at very long residence times of the reactants of 10 to 90 min. The catalyst solution is recycled after distilling off water and methacrolein. Since only water and methacrolein are distilled off, any higher boiling by-products remain in the catalyst solution and, thereby, might interfere with the reaction of propionaldehyde with formalin.

WO 2015/065610 A1, WO 2018/217961 A1, WO 2018/217962 A1, WO 2018/217963 A1 und WO 2018/217964 A1 disclose methods for producing methacrolein which is free of water and methanol and its use for the manufacturing of methacrylic acid and methyl methacrylate. A mixture of dimethyl amine and acetic acid is used as a catalyst. The reaction product is transferred into a phase separator at a temperature of less than 20 °C. The aqueous phase is recycled into a column. The overhead stream of the column comprises mainly methanol and methacrolein and can be further processed. The column furthermore comprises a side stream where a phase comprising mainly water is drawn. The side stream is gaseous and needs to be condensed before being further treated.

All of the above described processes require rather large amounts of catalyst for the preparation of methacrolein. Furthermore, high amounts of water are treated as waste water and, in cases in which the catalyst is recycled impurities are introduced into the reaction which negatively impact the yield of methacrolein. Furthermore, in some of the cases, the concentration of catalyst is increased which is also disadvantageous from a cost perspective.

Thus, there is need for a process which reduces the amount of waste water and which, at the same time, produces methacrolein of high purity.

### Objects

It was therefore an object of the present invention to provide a process which allows the production of methacrolein of high purity and which, at the same time, has a reduced amount of waste water.

### Solution

This object was solved by a process for preparing methacrolein comprising the following steps a) to g):
a) reacting in a first reactor formaldehyde and propionaldehyde in the presence of a homogeneous catalyst mixture, which comprises at least one acid and at least one base, to obtain a reaction mixture which comprises methacrolein, water, methanol, the homogeneous catalyst mixture and unconverted formaldehyde,
b) expanding the reaction mixture obtained in step a) in an expansion vessel to obtain a gas phase which comprises methacrolein, water and methanol, and a liquid phase which comprise water, the homogeneous catalyst mixture and unconverted formaldehyde,
c) introducing the gas phase obtained in step b) into a first condenser and condensing the gas phase in the first condenser to obtain a first condensed phase,
d) separating the first condensed phase obtained in step c) in a first phase separator to obtain an organic phase which comprises methacrolein and an aqueous phase which comprises water, residuals of methacrolein and methanol,
e) introducing the aqueous phase obtained in step d) into a first distillation column, wherein the first distillation column comprises at least one column internal selected from the group consisting of trays and packing materials, and wherein the aqueous phase is introduced into the first distillation column at a first feeding point which lies above the at least one column internal
f) introducing the liquid phase obtained in step b) into the first distillation column wherein the liquid phase is introduced into the first distillation column at a second feeding point which lies below the at least one column internal,
g) distilling the aqueous phase and the liquid phase in the first distillation column to obtain a first overhead stream and a first bottom stream.

It has surprisingly been found that the inventive process allows a reduction of the amount of homogeneous catalyst mixture over the lifetime of the process. Thus, the inventive process is more cost efficient and also more sustainable.

With regard to known processes the yield of methacrolein obtained in the synthesis could also be increased.

The amount of waste water can be reduced and, thereby, a significant amount of energy can be saved.

The expansion vessel of step b) is advantageous as the reaction mixture does not have to be cooled in total. Furthermore, the cooling is a result of the expansion and, thus, no additional energy expenditure or apparatus complexity is necessary for cooling. Thus, energy is saved by the inventive process. The combination of the expansion vessel with the first distillation column results in a smaller first distillation column than without the expansion vessel. This combination is furthermore advantageous since the first bottom stream of the first distillation column is essentially free of methacrolein. The organic phase obtained in step c) is sufficiently pure to be used in subsequent process steps, such as for example a direct oxidative esterification.

It has furthermore surprisingly been found that if, in a preferred embodiment, the flow ratio of the aqueous phase of the phase separator into the first distillation column to the liquid phase from the expansion vessel into the first distillation column is in the range from 0.15 to 0.5, preferably in the range from 0.2 to 0.4, the amount of amines, in particular resulting from the catalyst mixture which preferably comprises dimethylamine, can be reduced as well as the amount of formaldehyde, which both are comprised in the organic phase.

In process step a) in a first reactor formaldehyde and propionaldehyde are reacted in the presence of a homogeneous catalyst mixture. The homogeneous catalyst mixture comprises at least one acid and at least one base. A reaction mixture is obtained which comprises methacrolein, water, methanol, the homogeneous catalyst mixture and unconverted formaldehyde.

As first reactor any reactor which allows the reaction of formaldehyde and propionaldehyde in the presence of a homogeneous catalyst mixture is suitable. Preferably, the first reactor is a tubular reactor or a plate reactor. Those reactors are known for the reaction according to step a) and are, for example, disclosed in EP 3 194 355 B1.

Process step a) may be carried out at any temperature and at any pressure suitable for the reaction. Preferably, the temperature is in the range 110 °C to 180 °C, preferably in the range from 120 °C to 180 °C and particularly preferably in the range from 125 °C to 170 °C.

The pressure within the first reactor is preferably greater than the boiling pressure of the reaction mixture at the temperature at which the reaction is carried out. Thus, the pressure within the first reactor is preferably in the range from 10 bar to 50 bar, particularly preferably in the range from 20 bar to 50 bar.

Thus, the reaction in step a) is preferably carried out in liquid phase.

Formaldehyde is typically reacted as pure formaldehyde or as solution. Preferably, formaldehyde is reacted as aqueous solution. An aqueous solution of formaldehyde is also known as formalin. Preferably, the aqueous solution of formaldehyde comprises in the range from 35 wt.-% to 60 wt.-% of formaldehyde, based on the total weight of the aqueous solution. It is clear to the skilled person that the concentration of formaldehyde is based on the aqueous solution before it has entered the first reactor and, in particular, before the reaction of step a) has been started. During the reaction in step a), the concentration may change. The aqueous solution may furthermore comprise in the range from 0.5 wt.-% to 1 wt.-% methanol, based on the total weight of the aqueous solution. The aqueous solution may furthermore comprises traces of formic acid, such as about 100 wt.-ppm formic acid based on the total weight of the aqueous solution.

Propionaldehyde may be reacted as pure propionaldehyde or as solution, preferably, propionaldehyde is reacted as pure propionaldehyde. "Pure propionaldehyde" in this context means that the amount of water comprised in propionaldehyde is at most 5 wt.-% based on the total amount of water and propionaldehyde. It is clear to the skilled person that the water content is based on the propionaldehyde before it has entered the first reactor and, in particular, before the reaction of step a) has been started. During the reaction in step a), the concentration may change. Propionaldehyde may furthermore comprise impurities in an amount in the ppm range, such as propionic acid.

The molar ratio of propionaldehyde to formaldehyde before the reaction of step a) is for example in the range from 0.75 to 1, preferably in the range from 0.85 to 0.99 and particularly preferably in the range of 0. 94 to 0.98.

The reaction of step a) is carried out in the presence of a homogeneous catalyst mixture. "homogeneous" in this context means that the catalyst mixture and the reactants, formaldehyde and propionaldehyde, are in the same phase, preferably in the liquid phase, when added to the first reactor. It is possible that during the reaction or after the reaction the phases may differ, e.g. a two phase mixture may be obtained.

The homogeneous catalyst mixture comprises at least one acid and at least one base.
"at least one acid" means precisely one acid as well as a mixture of two or more acids. Precisely one acid is preferred. It is clear to the skilled person that "precisely one acid" refers to the catalyst mixture before the reaction of step a) has been carried out. It is possible that during the reaction further acids form. It is furthermore clear to the skilled person that further acids may be comprised in the first reactor. Those acids may for example be acids comprised in the formaldehyde and/or the propionaldehyde, such as formic acid and/or propionic acid.
"at least one base" means precisely one base as well as a mixture of two or more bases. As such, precisely one base is preferred. It is clear to the skilled person that "precisely one base" refers to the catalyst mixture before the reaction of step a) has been carried out. It is possible that during the reaction further bases form. For example, if the at least one base is or comprises dimethyl amine, during the reaction in step a) in general trimethylamine forms. Furthermore, the skilled person knows that traces of further bases may be comprised in the at least one base. For example, the at least one base may comprise about 40 wt.-ppm of monomethanolamine if, in a preferred embodiment, the at least one base is dimethylamine.

The at least one acid is generally any inorganic acid or organic mono-, di-, or polycarboxylic acid, preferably an organic monocarboxylic acid. Particularly preferably, the at least one acid is an aliphatic monocarboxylic acid, in particular at least one acid selected from the group consisting of formic acid, acetic acid and propionic acid. Acetic acid is particularly preferred.

The at least one base is in general an organic base. Suitable organic bases are known as such. Preferably, the at least one base is an organic amine, particularly preferably a secondary organic amine. Secondary organic amines are known as such and are preferably selected from the group consisting of dimethylamine, diethylamine, methylethylamine, methylpropylamine, dipropylamine, dibutylamine, di-isobutylamine, methylisopropylamine, methylisobutylamine, methyl-sec-butylamine, methyl(2-methylpentyl)amine, methyl(2-ethylhexyl)amine, pyrrolidine, piperidine, morpholine, N-methylpiperazine, N-hydroxyethylpiperazine, piperazine, hexylmethyleneimine, diethanolamine, methylethanolamine, methylcyclohexylamine, methylcyclopentylamine, and dicyclohexylamine. Particular preference is given to dimethylamine as at least one base.

It is, therefore, preferred according to the present invention that the homogeneous catalyst mixture comprises acetic acid and dimethylamine.

It is preferred that the homogeneous catalyst mixture comprises in the range from 1 mol to 1.5 mol, preferably in the range from 1.1 mol to 1.5 mol, and particularly preferably in the range from 1.1 mol to 1.4 mol of the at least one acid based on one mole of the at least one base. The ratio of the equivalents of the at least one base to the at least one acid and the specific choice thereof is preferably selected such that, measured at 20 °C at standard pressure in the first reactor prior to the reaction a pH of 3.0 to 7.0, preferably of 3.5 to 6.5 results.

The molar ratio of the at least one base to propionaldehyde before the reaction of step a) is carried out is for example in the range from 0.03 to 0.15, preferably in the range from 0.05 to 0.1 and particularly in the range from 0.06 to 0.09.

The water content in the first reactor before the reaction of step a) is carried out is for example in the range from 20 wt.-% to 80 wt.-%, preferably in the range from 30 wt.-% to 70 wt.-% and particularly preferably in the range from 40 wt.-% to 60 wt.-%, based on the total weight of all components in the first reactor before the reaction of step a).

A preferred embodiment of process step a) is for example disclosed in EP 3 194 355 B1, the content of which is incorporated by reference herein.

During the reaction in step a) methacrolein is formed. Thus in step a) a reaction mixture is obtained, which comprises methacrolein, water, methanol, the homogeneous catalyst mixture and unconverted formaldehyde.

Methanol, which is comprised in the reaction mixture obtained in step a) may for example result from an aqueous solution of formaldehyde which is reacted in step a).

For example, the reaction mixture comprises in the range from 2 to 60 wt.-%, preferably in the range from 5 to 40 wt.-% of methacrolein, based on the total weight of the reaction mixture.

For example, the reaction mixture comprises in the range from 40 to 95 wt.-%, preferably in the range from 50 to 90 wt.-% of water, based on the total weight of the reaction mixture.

For example, the reaction mixture comprises in the range from 0.5 to 10 wt.-%, preferably in the range from 0.7 to 6 wt.-% of the at least one base comprised in the homogeneous catalyst mixture, based on the total weight of the reaction mixture.

For example, the reaction mixture comprises in the range from 1 wt.-% to 18 wt.-%, preferably in the range from 2 wt.-% to 12 wt.-% of the at least one acid comprised in the homogeneous catalyst mixture, based on the total weight of the reaction mixture.

For example, the reaction mixture comprises in the range from 0.01 to 0.3 wt.-%, preferably in the range from 0.08 to 0.28 wt.-% of methanol, based on the total weight of the reaction mixture.

For example, the reaction mixture comprises in the range from 0.05 to 1 wt.-%, preferably in the range from 0.1 to 0.5 wt.-% of unconverted formaldehyde, based on the total weight of the reaction mixture.

The reaction mixture may comprise further components, such as di-methacrolein (DIMAL), trimethylamine, oligomers of methacrolein, stabilizers, such as TEMPOL and other by-products which may form during the reaction. Trimethylamine generally is formed from dimethylamine if the at least one base comprises dimethylamine. For example DIMAL, oligomers and stabilizers are so called high-boilers. The term "high-boilers" encompasses compounds which have a higher boiling point than methacrolein.

The reaction mixture exiting the first reactor has a temperature of at most 180 °C, preferably in the range from 150 °C to 180 °C, particularly preferably in the range from 155 °C to 170 °C.

Thus, preferred is also a process, in which the reaction mixture exiting the first reactor has a temperature in the range of from 150 °C to 180 °C.

The reaction mixture preferably has a pressure in the range from 15 bar (absolute) to 100 bar (absolute), particularly preferably in the range from 22 bar (absolute) to 50 bar (absolute) when exiting the first reactor, provided that it remains liquid at the selected temperature.

In step b), the reaction mixture obtained in step a) is expanded in an expansion vessel. Thereby a gas phase is obtained which comprises methacrolein, water and methanol and a liquid phase is obtained which comprises water, the homogeneous catalyst mixture and unconverted formaldehyde.

The expansion vessel typically has an inner temperature in the range from 50 °C to 150 °C, preferably in the range from 70 °C to 100 °C. The expansion vessel typically has an inner pressure in the range from 900 mbar to 1.1 bar.

The expansion of the reaction mixture in step b) causes a fraction of the reaction mixture to go into the gas phase which gives the gas phase obtained in step b).

The gas phase comprises in general in the range from 19 wt.-% to 95 wt.-%, preferably in the range from 29 wt.-% to 90 wt.-% of methacrolein, based on the total weight of the gas phase.

The gas phase comprises in general in the range from 4 wt.-% to 80 wt.-%, preferably in the range from 9 wt.-% to 70 wt.-% of water, based on the total weight of the gas phase.

The gas phase comprises in general in the range from 0.05 wt.-% to 0.5 wt.-%, preferably in the range from 0.1 wt.-% to 0.4 wt.-% of methanol, based on the total weight of the gas phase.

The gas phase may comprise further components which were also comprised in the reaction mixture, such as the homogeneous catalyst mixture, stabilizers and DIMAL.

Preferably, the gas phase comprises additionally amines. Amines which may be comprised in the gas phase are in particular amines which may be comprised as the at least one base of the homogeneous catalyst mixture or by-products formed therefrom. Thus, amines which may be comprised in the gas phase are in particular dimethylamine and/or trimethylamine.

For example, the gas phase may comprise up to 1500 wt.-ppm amines, based on the total weight of the gas phase.

The gas phase may furthermore comprise additionally the at least one acid which is comprised in the homogeneous catalyst mixture.

For example, the gas phase may comprise in the range from 0.01 wt.-ppm to 0.25 wt.-ppm, preferably in the range from 0.05 wt.-ppm to 0.15 wt.-ppm of the at least one acid, based on the total weight of the gas phase.

The liquid phase obtained in step b) comprises water, the homogeneous catalyst mixture and unconverted formaldehyde.

The liquid phase comprises in general in the range from 55 wt.-% to 97 wt.-%, preferably in the range from 70 wt.-% to 95 wt.-% of water, based on the total weight of the liquid phase.

The liquid phase comprises in general in the range from 2 wt.-% to 22 wt.-%, preferably in the range from 4 wt.-% to 18 wt.-% of the at least one acid which was comprised in the homogeneous catalyst mixture, based on the total weight of the liquid phase.

The liquid phase comprises in general in the range from 1 wt.-% to 12 wt.-%, preferably in the range from 2 wt.-% to 10 wt.-% of the at least one base which was comprised in the homogeneous catalyst mixture or a reaction product of the at least one base, based on the total weight of the liquid phase. A reaction product of the at least one base is, for example in the case of dimethylamine as at least one base monomethylamine and/or trimethylamine.

The liquid phase comprises in general in the range from 0.01 wt.-% to 2 wt.-%, preferably in the range from 0.2 wt.-% to 1 wt.-% of unconverted formaldehyde, based on the total weight of the liquid phase.

The liquid phase may comprise further components which were also comprised in the reaction mixture, such as high boilers like stabilizers, and DIMAL. The liquid phase may in particular comprise residuals of methacrolein. "Residuals of methacrolein" in this context means for example in the range from 0.5 wt.-% to 6 wt.-%, preferably in the range from 1.5 wt.-% to 4 wt.-% of methacrolein, based on the total weight of the liquid phase.

In step c) the gas phase obtained in step b) is introduced into a first condenser and condensed therein to obtain a first condensed phase. Preferably, the gas phase is condensed in the first condenser together with the first overhead stream obtained in step g) of the inventive process. Thus, the first condensed phase preferably comprises the gas phase and the first overhead stream in condensed form (i.e. in liquid phase). As first condenser any condenser known to the skilled person may be used. It is also possible to use more than one first condenser. For example, cascade of first condensers can be used. For example the first condenser of the cascade may use water having a temperature in the range from 20 °C to 40 °C and the second condenser of the cascade may use cooling brine having a temperature in the range from 3 °C to 10 °C.

In step d) the first condensed phase which is obtained in step c) is introduced into a first phase separator and separated therein. Thereby an organic phase which comprises methacrolein and an aqueous phase which comprises water, residuals of methacrolein and methanol is obtained.

The organic phase may additionally comprise water. For example, the organic phase comprises in the range from 1 wt.-% to 2 wt.-% of water, based on the total weight of the organic phase.

The organic phase may additionally comprise methanol. For example, the organic phase comprises in the range from 0.2 wt.-% to 0.5 wt.-% of methanol, based on the total weight of the organic phase.

Thus, the organic phase comprises in the range from 95 wt.-% to 98 wt.-% of methacrolein, based on the total weight of the organic phase.

Surprisingly, the obtained organic phase comprises only traces of amines. "Traces of amines" in the context of the organic phase for example means up to 0.01 wt.-% amines, based on the total weight of the organic phase. The organic phase may furthermore comprise traces of high boilers such as DIMAL.

The organic phase can be further reacted. For example, the organic phase may be transferred into a direct oxidative esterification (DOE) step. Such process is for example described in US 11,661,395, the content of which is hereby incorporated by reference.

The aqueous phase obtained in step d) comprises for example in the range from 85 wt.-% to 92 wt.-% of water, based on the total weight of the aqueous phase.

The aqueous phase obtained in step d) comprises for example in the range from 2 wt.-% to 4 wt.-% of methanol, based on the total weight of the aqueous phase.

The aqueous phase obtained in step d) comprises residuals of methacrolein. "residuals of methacrolein" in this context means in the range from 0.5 wt.-% to 8 wt.-% of methacrolein, based on the total weight of the aqueous phase.

The aqueous phase obtained in step d) preferably comprises trimethylamine. The trimethylamine may be formed from dimethylamine comprised in the homogeneous catalyst mixture. For example, the aqueous phase comprises in the range from 200 wt.-ppm to 3000 wt.-ppm of trimethylamine, based on the total weight of the aqueous phase.

The aqueous phase obtained in step d) preferably comprises acetic acid which was comprised in the homogeneous catalyst mixture. For example, the aqueous phase comprises in the range from 500 wt.-ppm to 3000 wt.-ppm of acetic acid, based on the total weight of the aqueous phase.

The aqueous phase preferably has a pH value of less than 7 at 20 °C. Thus, it is preferred that the aqueous phase is acidic. Preferably, the aqueous phase is acidic since it comprises an excess of the at least one acid comprised in the homogeneous catalyst mixture with regard to the at least one bases and by-products formed therefrom) comprised in the homogeneous catalyst mixture.

Preferred is therefore also a process in which the aqueous phase obtained in the phase separator has a pH value of less than 7 at 20 °C.

Suitable first phase separators for carrying out step d) are known to the skilled person, such as a decanter, a centrifuge and/or a coalescer. Preferred as first phase separator is a decanter.

In step e) the aqueous phase obtained in step d) is introduced into a first distillation column. The first distillation column comprises at least one column internal selected from the group consisting of trays, and packing materials. The aqueous phase is introduced into the first distillation column at a first feeding point which lies above the at least one column internal.

As first distillation column any column known to the skilled person which comprises at least one column internal is suitable.

"At least one column internal" means precisely one column internal as well as two or more column internals. Preferred are two or more column internals.

According to the present invention, the at least one column internal is selected from the group consisting of trays and packing materials. These column internals are known as such. Suitable trays are for example bubble-cap trays, sieve trays, cascade trays and valve trays. Suitable packing materials are for example Raschig rings or structured sheet metal (e.g. Sulzer Mellapak^{™}).

The first distillation column can be operated at standard pressure, for example, such that the bottom temperature of the column is somewhat above 100 °C. The temperature at the top of the distillation column is preferably in the range from 90 °C to 99 °C, preferably in the range from 91 °C to 97 °C.

In step f) the liquid phase obtained in step b) is introduced into the first distillation column at a second feeding point. The second feeding point lies below the at least one column internal.

Preferably, the first distillation column comprises two or more column internals and the second feeding point lies below a first column internal and above a second column internal.

Thus, preferred is a process in which the first distillation column comprises two or more column internals and wherein the second feeding point lies below a first column internal and above a second column internal.

It is preferable that the flow ratio of the aqueous phase which is obtained in the phase separator in step d) into the first distillation column (step e)) to the liquid phase which is obtained in step b) into the first distillation column (step f)) is in the range from 0.15 to 0.5, preferably in the range from 0.2 to 0.4.

Preferred is therefore also a process in which a flow ratio of the aqueous phase of the phase separator into the first distillation column to the liquid phase from the expansion vessel into the first distillation column is in the range from 0.15 to 0.5, preferably in the range from 0.2 to 0.4.

In step g) the aqueous phase and the liquid phase are distilled in the first distillation column to obtain a first overhead stream and a first bottom stream.

The first overhead stream comprises methacrolein, water and methanol.

For example, the first overhead stream comprises in the range from 15 wt.-% to 50 wt.-% of methacrolein, based on the total weight of the first overhead stream.

For example, the first overhead stream comprises in the range from 45 wt.-% to 80 wt.-% of water, based on the total weight of the first overhead stream.

For example, the first overhead stream comprises in the range from 4 wt.-% to 8 wt.-% of methanol, based on the total weight of the first overhead stream.

Additionally, the first overhead stream may comprise acetic acid and/ or traces of amines if the homogeneous catalyst mixture comprises acetic acid as the at least one acid and dimethylamine as the at least one base.

As outlined above, the first overhead stream is preferably introduced into the first condenser together with the gas phase obtained in step b).

Thus, preferred is a process in which the first overhead stream is obtained at the top of the first distillation column and is introduced into the first condenser.

The first bottom stream comprises unreacted formaldehyde, water, the homogeneous catalyst mixture and trimethylamine.

The first bottom stream is obtained at the bottom of the first distillation column. Preferably, the first bottom stream has a water content of at least 80 wt.-%, based on the total weight of the first bottom stream.

Preferred is, therefore, also a process in which the first bottom stream is obtained at the bottom of the first distillation column and wherein the first bottom stream has a water content of at least 85 wt.-%, based on the total weight of the first bottom stream.

A water content of at least 80 wt.-% in the first bottom stream is particularly advantageous since this reduces the risk of polymerization of MAL in the phase separator. If the water content is below 80 wt.-% the first overhead stream might become alkaline and result in polymerization in the first phase separator.

For example, the first bottom stream comprises in the range from 0.05 wt.-% to 2 wt.-% of unreacted formaldehyde, based on the total weight of the first bottom stream.

For example, the first bottom stream comprises in the range from 2 wt.-% to 20 wt.-% of the at least one acid comprised in the homogeneous catalyst mixture, based on the total weight of the first bottom stream.

For example, the first bottom stream comprises in the range from 1 wt.-% to 10 wt.-% of the at lease one base comprised in the homogeneous catalyst mixture, based on the total weight of the first bottom stream.

For example, the first bottom stream comprises in the range from 0.1 wt.-% to 0.6 wt.-% of trimethylamine, based on the total weight of the first bottom stream.

The first bottom stream may comprise traces of methacrolein.

A first part of the first bottom stream may be recycled into the first reactor. A second part of the first bottom stream may be introduced into a boil out stage. In the boil out stage a gas phase and an aqueous phase are obtained. The gas phase comprises water and methacrolein, the aqueous phase comprises mainly water and homogeneous catalyst mixture.

It is also possible that the second part of the first bottom stream is at least partially discarded, for example into a thermal oxidizer.

It is also possible that the second part of the first bottom stream is introduced into a third distillation column. Alternatively, the aqueous phase of the boil out stage may be introduced into the third distillation column. In this third distillation column, a third overhead stream and a third bottom stream are obtained. The third overhead stream comprises water, the third bottom stream comprises the homogeneous catalyst mixture. Thus, in the third distillation column, the homogeneous catalyst mixture is recovered. The third distillation column is therefore also called "catalyst concentration unit".

Therefore, a process is preferred, in which a first part of the first bottom stream is recycled into the first reactor and a second part of the first bottom stream is introduced into a third distillation column in which a third overhead stream comprising water and a third bottom stream comprising the homogeneous catalyst mixture is obtained.

The third bottom stream may be at least partially recycled into the first reactor. It is advantageous and therefore preferred that about 10 % to 30% of the third bottom stream are discarded, for example in a thermal oxidizer.

It is preferable that the ratio of the first part of the first bottom stream to the second part of the first bottom stream is adjusted in a way that the overall amount of water in the feed to the first reactor in step a) does not exceed 56 wt.-%, based on the total weight of the feed. It is clear to the skilled person that the feed includes all components introduced into the first reactor, such as formaldehyde, propionaldehyde, the homogeneous catalyst mixture etc.

The third bottom stream comprises in the range from 10 wt.-% to 30 wt.-% of the at least one acid comprised in the homogeneous catalyst mixture, based on the total weight of the third bottom stream.

The third bottom stream comprises in the range from 2 wt.-% to 19 wt.-% of the at least one base comprised in the homogeneous catalyst mixture, based on the total weight of the third bottom stream.

The third bottom stream generally furthermore comprises in the range from 30 wt.-% to 80 wt.-% water, based on the total weight of the third bottom stream.

Preferred is therefore also a process in which the third bottom stream obtained in the third distillation column comprises in the range from 30 wt.-% to 80 wt.-% water, based on the total weight of the third bottom stream.

The third bottom stream may furthermore comprise high boilers as well as trimethylamine.

The third bottom stream may be recycled into the first reactor. This is advantageous since the homogeneous catalyst mixture can be reused which makes the inventive process particularly economic.

It is therefore preferred that the third bottom stream obtained in the third distillation column is recycled into the first reactor.

The third overhead stream which is obtained in the third distillation column, after condensation has preferably a pH value at 20 °C of more than 7. Most of the trimethylamine which was comprised in the second part of first bottom stream is obtained in the third overhead stream. The pH value of the third overhead stream essentially depends on the amount of trimethylamine obtained in the third overhead stream.

Thus, preferred is a process in which the third overhead stream obtained in the third distillation column after condensation has a pH value at 20 °C of more than 7.

The third overhead stream is preferably at least partially introduced into at least one of a biological waste water treatment and a thermal oxidizer. These methods are known as such. It is preferred that the third overhead stream is directly introduced into at least one of a biological waste water treatment and a thermal oxidizer. "Directly introduced" in this context means that the third overhead stream is not condensed but introduced into at least one of a biological waste water treatment and a thermal oxidizer in gaseous form.

In a preferred embodiment of the present invention, a side stream is removed from the first distillation column. The side stream may be in gaseous or liquid form, preferably in liquid form. Thus, preferably, a liquid side stream is removed from the first distillation column.

The side stream, preferably the liquid side stream, comprises mostly water, traces of methacrolein, unconverted formaldehyde, trimethylamine and the at least one acid of the homogeneous catalyst mixture.

Within the context of the side stream, the term "traces of methacrolein" means in the range from 500 wt.-ppm to 1500 wt.-ppm methacrolein, based on the total weight of the side stream.

The side stream may comprise in the range from 200 wt.-ppm to 3000 wt.-ppm of acetic acid, based on the total weight of the side stream.

The side stream may comprise in the range from 100 wt.-ppm to 2000 wt.-ppm of trimethyl amine, based on the total weight of the side stream.

The side stream may comprise in the range from 500 wt.-ppm to 5000 wt.-ppm of unconverted formaldehyde, based on the total weight of the side stream.

In general, the side stream comprises in the range from 98 wt.-% to 99.5 wt.-% water, based on the total weight of the side stream.

The side stream, in particular the liquid side stream is preferably removed at a first extraction point. The first extraction point may, for example lie below the first feeding point and above the second feeding point.

Preferred is therefore also a process in which a side stream is removed from the first distillation column wherein the side stream is removed at a first extraction point, which lies below the first feeding point and above the second feeding point, wherein the side stream comprises mostly water, traces of methacrolein, unconverted formaldehyde and the at least one acid of the homogeneous catalyst mixture.

The obtained side stream may at least partially be recycled into the first distillation column.

Preferred is therefore also a process in which the side stream is at least partially recycled into the first distillation column.

If a side stream is removed from the first distillation column, the first distillation column preferably comprises the following components in the given order (from top to bottom):
- first overhead stream outlet,
- liquid distributor for aqueous phase,
- first column internal,
- liquid collector and side stream outlet
- Liquid distributor for the liquid phase
- inlet for parts of the side stream (may be at the same height as the liquid distributor for the liquid phase)
- one or more column internals
- bottom of column (evaporator)

Additionally or alternatively to an at least partial recycling, the side stream may at least partially be introduced into at least one of a biological waste water treatment and a thermal oxidizer. Biological waste water treatment and thermal oxidizers are known as such.

In a further embodiment, the obtained side stream may at least partially be introduced into a second distillation column. By distilling the side stream, a second overhead stream and a second bottom stream are obtained. The second overhead stream may at least partially be transferred into the first condenser.

The second bottom stream may be introduced into at least one of a biological waste water treatment and a thermal oxidizer. Biological waste water treatment and thermal oxidizers are known as such.

It is therefore preferred that the side stream is introduced into a second distillation column to obtain a second overhead stream and a second bottom stream, wherein the second overhead stream is at least partially transferred into the first condenser.

The second overhead stream generally comprises water, methacrolein and trimethylamine.

The second bottom stream generally comprises water, unreacted formaldehyde and acetic acid.

### Reference Numbers

- A: first reactor
- B: expansion vessel
- C: first condenser
- D: first phase separator
- E: first distillation column
- F: column internal
- Fa: first column internal
- Fb: second column internal
- G: first feeding point
- H: second feeding point
- I: first extraction point
- J: third distillation column

- 1: formaldehyde
- 2: propionaldehyde
- 3: homogeneous catalyst mixture
- 3a: at least one acid
- 3b: at least one base
- 4: reaction mixture
- 5: gas phase
- 6: liquid phase
- 7: first condensed phase
- 8: organic phase
- 9: aqueous phase
- 10: first overhead stream
- 11: first bottom stream
- 11a: first part of the first bottom stream
- 11b: second part of the first bottom stream
- 12: side stream
- 13: third bottom stream
- 13a: first part of the third bottom stream
- 13b: second part of the third bottom stream
- 14: third overhead stream

### Figures

In the figures, identical reference numbers have the same meaning and refer to the same parts of the manufacturing process.

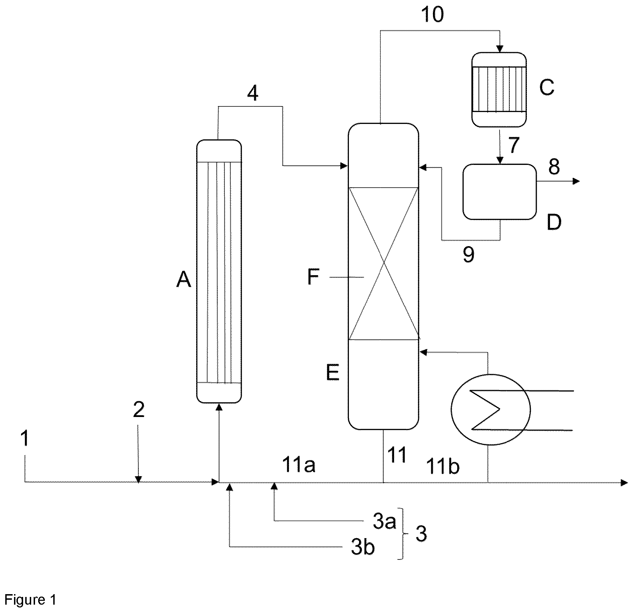

Figure 1 shows a process for manufacturing methacrolein according to the prior art. According to this figure formaldehyde 1 and propionaldehyde 2 are introduced into a first reactor A together with a homogeneous catalyst mixture 3 comprising at least one acid 3a and at least one base 3b. Formaldehyde 1 and propionaldehyde 2 are reacted in the first reactor A in the presence of the homogeneous catalyst mixture 3 to obtain a reaction mixture 4. The reaction mixture 4 is introduced on top of a first distillation column E. The first distillation column E comprises a column internal F. The reaction mixture 4 is added above the column internal F. In the first distillation column E the reaction mixture 4 and an aqueous phase 9 are distilled to obtain a first overhead stream 10 and a first bottom stream 11. The first overhead stream 10 is condensed in a first condenser C to obtain a first condensed phase 7. The first condensed phase 7 is introduced into a first phase separator D. In the first phase separator D an organic phase 8 and the aqueous phase 9 are obtained. The aqueous phase 9 is added on top of the first distillation column E above the column internal F. The first bottom stream 11 is divided into a first part of the first bottom stream 11a and a second part of the first bottom stream 11b. The first part of the first bottom stream 11a is recycled into the first reactor A. The second part of the first bottom stream 11b is partially returned into the first distillation column E and partially discarded, for example into a thermal oxidizer.

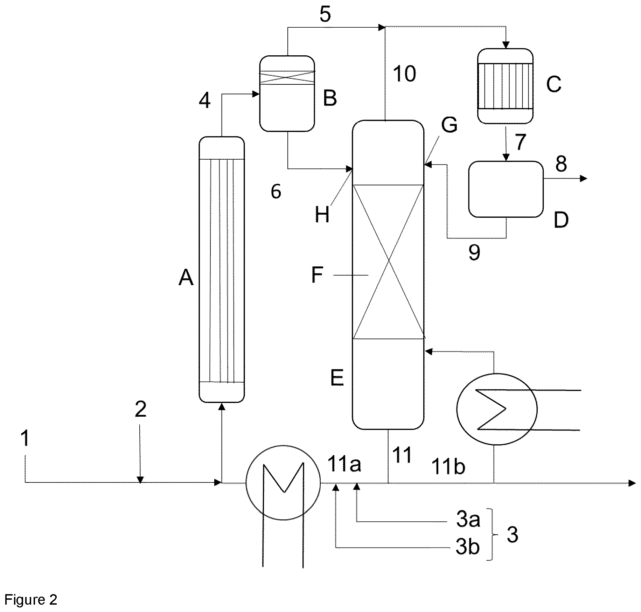

Figure 2 shows another process for manufacturing methacrolein according to the prior art. In the following, only the differences with regard to figure 1 are described. The reaction mixture 4 obtained in the first reactor 4 is introduced into an expansion vessel B. In the expansion vessel B a gas phase 5 and a liquid phase 6 are obtained. The liquid phase 6 is introduced into the first distillation column E at a second feeding point H which lies above the column internal F. The gas phase 5 is added into the first condenser C together with the first overhead stream 10. The aqueous phase 9 obtained in the first phase separator D is introduced into the first distillation column E at a first feeding point G.

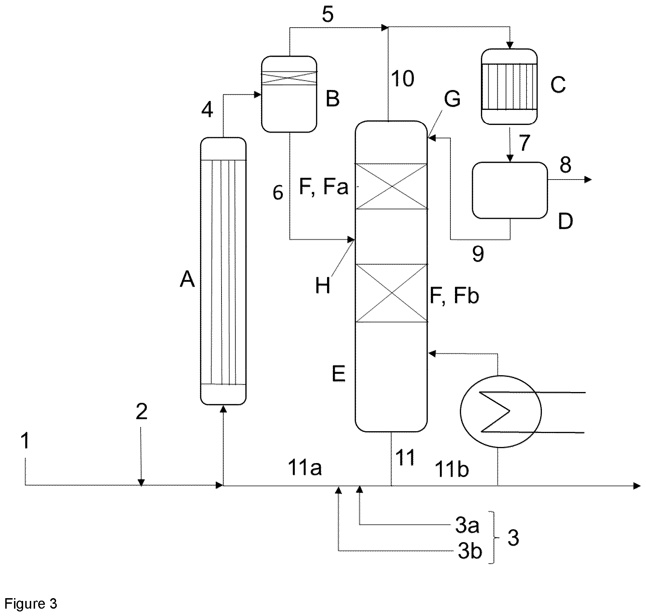

Figure 3 shows a process for manufacturing methacrolein according to a first embodiment of the present invention. In the following only differences with regard to figure 2 are described. The first distillation column E shown in figure 3 comprises a first column internal Fa and a second column internal Fb. The second feeding point H of the liquid phase 6 lies below the first column internal Fa and above the second column internal Fb. The first feeding point G of the aqueous phase 9 lies above the first column internal Fa.

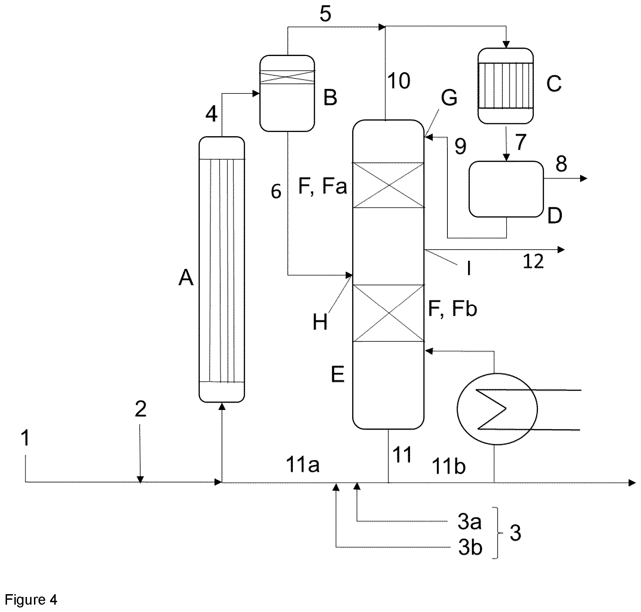

Figure 4 shows a process for manufacturing methacrolein according to a second embodiment of the present invention. In the following only differences with regard to figure 3 are described. At a first extraction point I a side stream 12 is removed from the first distillation column E. The first extraction point I lies below the first column internal Fa and above the second column internal Fb. The first extraction point I also lies above the second feeding point H.

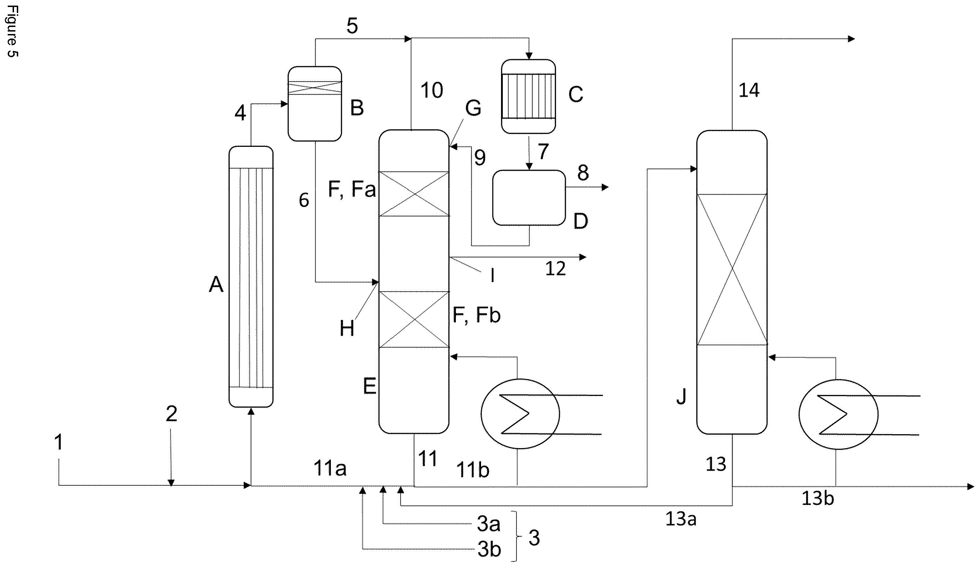

Figure 5 shows a process for manufacturing methacrolein according to a third embodiment of the present invention. In the following, only differences with regard to figure 4 are described. The second part of the first bottom stream 11b is partially introduced in a third distillation column J. It is preferably introduced at the top of the third distillation column J. The second part of the first bottom stream 11b is distilled in the third distillation column J to obtain a third overhead stream 14 and a third bottom stream 13. A first part of the third bottom stream 13a is recycled into the first reactor A and a second part of the third bottom stream 13b is partially recycled into the third distillation column J and partially discarded, for example in a thermal oxidizer. The third overhead stream 14 is also discarded, for example into a thermal oxidizer.

### Examples

### Comparative Example 1:

10.9 kg/h propionaldehyde (Oxea Oberhausen) and 10.4 kg/h formaldehyde (55% in water) were mixed in a static mixer and preheated to a temperature of approx. 126 °C in an oil-heated coil heater. Approx. 20.6 kg/h of the first bottom stream from the first distillation column was mixed with 0.32 kg/h acetic acid and 0.523 kg/h dimethylamine (40% water) and the homogeneous catalyst mixture thereby obtained was also preheated to 126°C in an oil-heated coil. Subsequently, the aldehyde solution comprising formaldehyde (55 wt.-% in water) and propionaldehyde and the homogeneous catalyst mixture were mixed in another static mixer and this mixture was fed into an oil-heated tube reactor (first reactor) with a diameter of 4 mm and a length of approx. 8 m. The oil flow temperature was 156.7°C. The pressure in the first reactor was set to 30 bar by means of a valve directly downstream of the first reactor. The obtained reaction mixture was processed according to option 1: The reaction mixture was expanded at a temperature of approx. 158 °C onto the head of the first distillation column (ID = 100 mm). Below the addition point, a Sulzer Melapak^{™} packing (length = 2.5 m) and another Sulzer Melapak^{™} packing (length = 3.5 m) were installed. The gas phase obtained in the expansion was fed into a condenser.

The first overhead stream from this first distillation column was condensed in a first condenser and the condensed phase was fed into a decanter (first phase separator). Approximately 13.3 kg/h of methacrolein were obtained as organic phase. The aqueous phase obtained in the first phase separator was returned to the head of the first distillation column at a mass flow rate of 7.3 kg/h.

The bottom of the first distillation column was heated by means of forced circulation (circulation approx. 600 kg/h) and a shell-and-tube heat exchanger with approx. 5.5 kg/h 11 bar of steam. The first distillation column was operated at normal pressure. Approximately 8.8 kg/h of the first bottom stream was discharged as waste water and approximately 20.5 kg/h of the same first bottom stream was returned to the first reactor for recycling.

Conversion was greater than 99.9%. The molar MAL selectivity was about 98.3 %.

### Comparative Example 2:

9.1 kg/h propionaldehyde (Oxea Oberhausen) and 8.7 kg/h formaldehyde (55% in water) were mixed in a static mixer and preheated to a temperature of approx. 123.4°C in an oil-heated coil heater. Approximately 22.3 kg/h of the first bottom stream from the first distillation column was mixed with 0.27 kg/h acetic acid and 0.44 kg/h dimethylamine (40% water) and the homogeneous catalyst mixture obtained was also preheated to 123.4°C in an oil-heated coil. Subsequently, the aldehyde solution comprising formaldehyde (55 % in water) and propionaldehyde and the homogeneous catalyst mixture were mixed in another static mixer and this mixture was fed into an oil-heated tube reactor (first reactor) with a diameter of 4 mm and a length of approx. 8 m. The oil flow temperature was 157°C. The pressure in the first reactor was set to 30 bar by means of a valve directly downstream of the first reactor. The reaction mixture was processed according to option 2: The reaction mixture was expanded into an expansion vessel at a temperature of approx. 157.4 °C. The temperature in the expansion vessel was approx. 79.3 °C. The liquid phase is fed from the flash vessel (approx. 27.9 kg/h) to the head of the first distillation column. The feed was added above a Sulzer Melapak^{™} pack (length = 2.5 m) and a Sulzer Melapak^{™} pack (length = 3.5 m).

The first overhead stream from this first distillation column was combined with the gas phase from the expansion vessel, condensed in a first condenser and the condensed phase was fed into a decanter (first phase separator). Approximately 11.1 kg/h of methacrolein were obtained as organic phase. The aqueous phase obtained in the first phase separator was returned to the head of the first distillation column with a mass flow rate of 5.3 kg/h above a Sulzer Melapak^{™} pack (length = 2.5 m) and a Sulzer Melapak^{™} pack (length = 3.5 m). The ratio of aqueous phase to liquid phase fed into the first distillation column was thus 0.19.

The first bottom stream of the first distillation column was heated by means of forced circulation (circulation approx. 600 kg/h) and a shell-and-tube heat exchanger with approx. 5 kg/h of steam (11 bar). The first distillation column was operated at normal pressure. Approximately 7.5 kg/h of the first bottom stream was discharged as wastewater and approximately 22.3 kg/h of the first bottom stream drain was returned to the first reactor for recycling.

Conversion was greater than 99.9%. The molar methacrolein (MAL) selectivity was approximately 98.3%.

### Example 3:

10.9 kg/h propionaldehyde (Oxea Oberhausen) and 10.4 kg/h formaldehyde (55% in water) were mixed in a static mixer and preheated to a temperature of approx. 126 °C in an oil-heated coil heater. Approximately 20.6 kg/h of first bottom stream from the first distillation column was mixed with 0.32 kg/h acetic acid and 0.523 kg/h dimethylamine (40% water) and the homogeneous catalyst mixture obtained was preheated to 126°C in an oil-heated coil. Subsequently, the aldehyde solution comprising formaldehyde (55 % in water) and propionaldehyde and the homogeneous catalyst mixture were mixed in another static mixer and this mixture was fed into an oil-heated tube reactor (first reactor) with a diameter of 4 mm and a length of approx. 8 m. The oil flow temperature was 156.7°C. The pressure in the first reactor was set to 30 bar by means of a valve directly downstream of the first reactor. The reaction mixture was processed according to option 3: The reaction mixture was expanded at a temperature of approx. 157.8 °C into an expansion vessel. The temperature in the expansion vessel was about 79.2°C. The liquid phase from the expansion vessel (approx. 27.4 kg/h) was fed into the middle of a first distillation column (ID = 100 mm) with a Sulzer Melapak^{™} pack (length = 2.5 m) above the addition point of the liquid phase and a Sulzer Melapak pack (length = 3.5 m) below the addition point.

The first overhead stream from this first distillation column was combined with the gas phase from the expansion vessel, condensed in a first condenser and the condensed phase was fed into a decanter (first phase separator). Approximately 13.3 kg/h of methacrolein were obtained as organic phase. The aqueous phase obtained in the first phase separator was returned to the head of the first distillation column with a mass flow of 6.6 kg/h above the Sulzer Melapak^{™} pack (length = 2.5 m) and the Sulzer Melapak^{™} pack (length = 3.5 m). The ratio of aqueous phase to liquid phase fed into the first distillation column was thus 0.24.

The first bottom stream of the first distillation column was heated by means of forced circulation (circulation approx. 600 kg/h) and a shell-and-tube heat exchanger with approx. 5.4 kg/h 11 bar of steam. The first distillation column was operated at normal pressure. Approximately 8.8 kg/h of the first bottom stream was discharged as waste water and approximately 20.6 kg/h of the same first bottom stream was returned to the first reactor for recycling.

Conversion was greater than 99.9%. The molar MAL selectivity was about 98.3 %.

### Example 4:

7.3 kg/h propionaldehyde (Oxea Oberhausen) and 7 kg/h formaldehyde (55% in water) were mixed in a static mixer and preheated to a temperature of approx. 127.4°C in an oil-heated coil heater. Approximately 14 kg/h of first bottom stream from the first distillation column was mixed with 0.22 kg/h acetic acid and 0.35 kg/h dimethylamine (40% water) and the homogeneous catalyst mixture obtained was also preheated to 127.4°C in an oil-heated coil. Subsequently, the aldehyde solution comprising formaldehyde (55 % in water) and propionaldehyde and the homogeneous catalyst mixture were mixed in another static mixer and this mixture was fed into an oil-heated tube reactor (first reactor) with a diameter of 4 mm and a length of approx. 8 m. The oil flow temperature was 154°C. The pressure in the first reactor was set to 30 bar by means of a valve directly downstream of the first reactor. The reaction mixture was processed according to option 3. The reaction mixture was expanded into an expansion vessel at a temperature of approx. 157°C. The temperature in the expansion vessel was approx. 79°C. The liquid phase from the expansion vessel (approx. 19.3 kg/h) was fed into the middle of the first distillation column (ID = 100 mm) with a Sulzer Melapak^{™} pack (length = 2.5 m) above the addition point (second feeding point) and a Sulzer Melapak^{™} pack (length = 3.5 m) below the second feeding point.

The first overhead stream from this first distillation column was combined with the gas phase from the expansion vessel, condensed in a first condenser and the condensed phase was fed into a decanter (first phase separator). Approximately 8.9 kg/h of methacrolein was obtained as organic phase. The aqueous phase obtained in the first phase separator was returned to the head of the first distillation column at a mass flow rate of 9 kg/h. The ratio of aqueous phase to liquid phase fed into the first distillation column was thus 0.46.

The first bottom stream of the first distillation column was heated by means of forced circulation (circulation approx. 600 kg/h) and a shell-and-tube heat exchanger with approx. 8.4 kg/h 11 bar of steam. The first distillation column was operated at normal pressure. Approx. 6.1 kg/h of the first bottom stream was discharged as wastewater and approx. 14 kg/h of the same first bottom stream was returned to the first reactor as recycling.

Conversion was greater than 99.9%. The molar methacrolein (MAL) selectivity was approximately 98.3%.

### Example 5:

7.3 kg/h propionaldehyde (Oxea Oberhausen) and 7.1 kg/h formaldehyde (55%) were mixed in a static mixer and preheated to a temperature of approx. 127.4°C in an oil-heated coil heater. Approximately 14 kg/h of first bottom stream from the first distillation column was mixed with 0.22 kg/h acetic acid and 0.35 kg/h dimethylamine (40% water) and the homogeneous catalyst mixture obtained was also preheated to 127.4°C in an oil-heated coil. Subsequently, the aldehyde solution comprising formaldehyde (55 % in water) and propionaldehyde and the homogeneous catalyst mixture were mixed in another static mixer and this mixture was fed into an oil-heated tube reactor (first reactor) with a diameter of 4 mm and a length of approx. 8 m. The oil flow temperature was 154°C. The pressure in the first reactor was set to 30 bar by means of a valve directly downstream of the first reactor. The reaction mixture was processed according to option 3. The reaction mixture was expanded into a expansion vessel at a temperature of approx. 157.5 °C. The temperature in the expansion vessel was approx. 77°C. The liquid phase from the expansion vessel (approx. 19.3 kg/h) was fed into the center of the first distillation column (ID = 100 mm) with a Sulzer Melapak^{™} packing (length = 2.5 m) above the addition point (second feeding point) and a Sulzer Melapak^{™} packing (length = 3.5 m) below the addition point.

The first overhead stream from this first distillation column was combined with the gas phase from the expansion vessel, condensed in a first condenser and the condensed phase was fed into a decanter (first phase separator). Approximately 8.9 kg/h of methacrolein was obtained as organic phase. The aqueous phase obtained in the first phase separator was returned to the head of the first distillation column at a mass flow rate of 5.2 kg/h. The ratio of aqueous phase to liquid phase fed into the first distillation column was thus 0.27.

The first bottom stream of the first distillation column was heated by means of forced circulation (circulation approx. 600 kg/h) and a shell-and-tube heat exchanger with approx. 5.3 kg/h 11 bar of steam. The first distillation column was operated at normal pressure. Approx. 6.2 kg/h of the first bottom stream was discharged as wastewater and approx. 14 kg/h of the same first bottom stream was returned to the first reactor as recycling.

Conversion was greater than 99.9%. The molar methacrolein (MAL) selectivity was approximately 98.3%.

### Example 6

8.8 kg/h propionaldehyde (Oxea Oberhausen) and 8.4 kg/h formaldehyde (55% in water) were mixed in a static mixer and preheated to a temperature of approx. 123°C in an oil-heated coil heater. Approximately 17 kg/h of first bottom stream from the first distillation column was mixed with 0.26 kg/h acetic acid and 0.42 kg/h dimethylamine (40% water) and the homogeneous catalyst mixture obtained was also preheated to 123°C in an oil-heated coil. Subsequently, the aldehyde solution comprising formaldehyde (55 % in water) and propionaldehyde and the homogeneous catalyst mixture were mixed in another static mixer and this mixture was fed into an oil-heated tube reactor (first reactor) with a diameter of 4 mm and a length of approx. 8 m. The oil flow temperature was 153.9°C. The pressure in the first reactor was set to 30 bar by means of a valve directly downstream of the first reactor. The reaction mixture was processed according to option 3. The reaction mixture was expanded into an expansion vessel at a temperature of approx. 157.4°C. The temperature in the expansion vessel was approx. 79.1°C. The liquid phase from the expansion vessel (approx. 23 kg/h) was fed into the middle of the first distillation column (ID = 100 mm) with a Sulzer Melapak^{™} pack (length = 2.5 m) above the addition point (second feeding point) and a Sulzer Melapak^{™} pack (length = 3.5 m) below the addition point.

The first overhead stream from this first distillation column was combined with the gas phase from the expansion vessel, condensed in a first condenser and the condensed phase was fed into a decanter (first phase separator). Approximately 10.7 kg/h of methacrolein was obtained as organic phase. The aqueous phase obtained in the first phase separator was returned to the head of the first distillation column at a mass flow rate of 5.2 kg/h. The ratio of aqueous phase to liquid phase fed into the first distillation column was thus 0.23.

The first bottom stream of the first distillation column was heated by means of forced circulation (circulation approx. 600 kg/h) and a shell-and-tube heat exchanger with approx. 4.7 kg/h 11 bar of steam. The first distillation column was operated at normal pressure. Approx. 7.3 kg/h of the first bottom stream was discharged as wastewater and approx. 17 kg/h of the same first bottom stream was returned to the first reactor as recycling.

Conversion was greater than 99.9%. The molar methacrolein (MAL) selectivity was approximately 98.3%.

### Example 7

10.9 kg/h propionaldehyde (Oxea Oberhausen) and 10.4 kg/h formaldehyde (55% in water) were mixed in a static mixer and preheated to a temperature of approx. 126 °C in an oil-heated coil heater. Approximately 20.6 kg/h of first bottom stream from the first distillation column was mixed with 0.27 kg/h acetic acid and 0.43 kg/h dimethylamine (40% water) and the homogeneous catalyst mixture obtained was also preheated to 126°C in an oil-heated coil. Subsequently, the aldehyde solution comprising formaldehyde (55 % in water) and propionaldehyde and the homogeneous catalyst mixture were mixed in another static mixer and this mixture was fed into an oil-heated tube reactor (first reactor) with a diameter of 4 mm and a length of approx. 8 m. The oil flow temperature was 155.7°C. The pressure in the first reactor was set to 30 bar by means of a valve directly downstream of the first reactor. The reaction mixture was processed according to option 4: The reaction mixture was expanded into an expansion vessel at a temperature of approx. 157.3°C. The temperature in the expansion vessel was approx. 79.3 °C. The liquid phase from the expansion vessel (approx. 30 kg/h) was fed into the middle of the first distillation column (ID = 100 mm) with a Sulzer Melapak^{™} pack (length = 2.5 m) above the addition point (second feeding point) and a Sulzer Melapak^{™} pack (length = 3.5 m) below the addition point (second feeding point).

Above the second feeding point and below the Sulzer Melapak^{™} pack (length = 2.5 m) there was a water collector in the first distillation column, which collects the liquid effluent of the aforementioned pack positioned above the second feeding point. A side stream of 1.5 kg/h was taken from this collector.

The first overhead stream from this first distillation column was combined with the gas phase from the expansion vessel, condensed in a first condenser and the condensed phase was fed into a decanter (first phase separator). Approximately 13.3 kg/h of organic phase rich in methacrolein was obtained. The aqueous phase obtained in the first phase separator was returned to the head of the first distillation column with a mass flow rate of 5.7 kg/h. The ratio of aqueous phase to liquid phase fed into the first distillation column was thus 0.19.

The first bottom stream of the first distillation column was heated by means of forced circulation (circulation approx. 600 kg/h) and a shell-and-tube heat exchanger with approx. 5.2 kg/h 11 bar of steam. The first distillation column was operated at normal pressure. Approximately 7.5 kg/h of the first bottom stream was discharged as wastewater and approximately 20.6 kg/h of the same first bottom stream was returned to the first reactor for recycling.

Conversion was greater than 99.9%. The molar methacrolein (MAL) selectivity was about 98.3 %.

The side stream contains about 1000 ppm methacrolein, 1800 ppm acetic acid, 850 ppm TMA, 140 ppm DMA and 3100 ppm formaldehyde. The losses via the side stream are small: 0.01 % methacrolein, 1 % acetic acid and 0.1 % formaldehyde are lost via the side stream. Approximately 5% of the TMA formed is expelled. The side stream contains approx. 14,000 ppm COD (Chemical Oxygen Demand). Compared to comparative example 1 and inventive example 3, 17% less catalyst is used.

The above described examples show significant differences in the purity of the organic phase obtained in the first phase separator. Table 1 shows the amount of by-products obtained in the organic phase

**Table 1:**

| | **Work-up Option** | **Reflux / Feed** | **ACOH** | **org. N** | **FO** |
|---|---|---|---|---|---|
| | | | **wt.-ppm** | **wt.-ppm** | **wt.-ppm** |
| Comp. Ex. 1 | 1 | | 630 | 4.3 | 500 |
| Comp. Ex. 2 | 2 | | 364 | 1.9 | 650 |
| Ex. 3 | 3 | 0.24 | 202 | 0.9 | 600 |
| Ex. 4 | 3 | 0.46 | 98 | 0.4 | 1600 |
| Ex. 5 | 3 | 0.27 | 140 | 0.6 | 1100 |
| Ex. 6 | 3 | 0.23 | 210 | 1.0 | 550 |
| Ex. 7 | 4 | 0.21 | 193 | 1.2 | 550 |

From table 1, it can be seen that the work-up according to option 1 (comparative example 1) results in high amounts of acetic acid (ACOH) in the obtained organic phase. The amount of organic nitrogen (DMA and TMA) is also particularly high. Nevertheless, the amount of unreacted formaldehyde (FO) is rather low.

The work-up according to option 2 (comparative example 2) results in lower amounts of ACOH and org. N, but it is still higher than in the inventive examples. Nevertheless, it shows the advantage of the use of a expansion vessel. The amount of FO is higher than in comparative example 1.

The work-up according to option 3 (Inventive examples 3 to 6) results in significantly reduced amounts of ACOH and org. N. It can also be seen that the amount of FO correlates with the reflux. The higher the reflux, the more FO is comprised in the organic phase.

If additionally a side stream is drawn (option 4, inventive example 7), ACOH and org. N are reduced. Also FO is almost as low as for comparative example 1 (option 1).

### Example 8

In a stainless steel pressure vessel equipped with an EKATO Phasejet and an EKATO Combijet agitator, 1 kg of catalyst comprising 0.91 wt% gold, 1.10 wt% cobalt, 2.7 wt% magnesium, 7.4 wt% Al₂O₃, 87.8 wt% SiO₂ (produced according to WO 2022/017755 A1, examples 1 and 2a) was dispersed in methanol whereby a solids concentration of 9% was achieved in the suspension. The suspension was pressed to 5 bar absolute at a temperature of 80 °C under nitrogen. Methacrolein and methanol were continuously dosed in a molar ratio of 1 to 4 in the inlet, as well as a stabilizer content of TEMPOL of 100 ppm. The reactor was simultaneously gassed with oxygen, so that the oxygen concentration in the reactor's exhaust gas was 4% by volume (explosion limit is 7.8% oxygen by volume). The inlet rate and thus residence time were adjusted so that the catalyst load was 10 mol methacrolein / kg catalyst x hr. The pH of the reaction was kept constant at 7 by adding a solution of 4% NaOH, 5.5% H₂O and 90.5% methanol. The reactor was periodically sampled and analyzed using GC. Each methacrolein batch was dosed over a period of 150 hours, with the first 50 hours excluded from the performance determination to ensure that the methacrolein of the previous batch was either consumed or displaced from the reactor system.

Table 2 shows the results of the reaction for the methacrolein (organic phase) obtained in the above-described examples.

**Table 2:**

| **org. Phase of** | **MAL-Work-Up Option** | **org. N** | **FO** | **MAL Conversion** |
|---|---|---|---|---|
| | | **ppm** | **ppm** | **[%]** |
| Comp. Ex. 2 | 2 | 2.0 | 650 | 75.8 |
| Ex. 3 | 3 | 0.9 | 600 | 76.7 |
| Ex. 4 | 3 | 0.4 | 1600 | 75.7 |
| Ex. 5 | 3 | 0.6 | 1100 | 77.6 |
| Ex. 6 | 3 | 1.0 | 550 | 77.8 |
| Ex. 7 | 4 | 1.1 | 550 | 77.4 |

As can be seen from table 2, an increased amount of org. N and/or FO in the organic phase results in lower conversion of MAL.

The table does not comprise results for comparative example 1. During the reaction of the organic phase from this example, the 15 mm metal sinter filter of the methacrolein feed line became clogged because of the high amount of organic nitrogen in the organic phase . Thus, the reaction had to be stopped to clean the filter and was not resumed due to the risk of a new clogging.

## Claims

1. A process for preparing methacrolein comprising the following steps a) to g):
a) reacting in a first reactor formaldehyde and propionaldehyde in the presence of a homogeneous catalyst mixture, which comprises at least one acid and at least one base, to obtain a reaction mixture which comprises methacrolein, water, methanol, the homogeneous catalyst mixture and unconverted formaldehyde,
b) expanding the reaction mixture obtained in step a) in an expansion vessel to obtain a gas phase which comprises methacrolein, water and methanol, and a liquid phase which comprise water, the homogeneous catalyst mixture and unconverted formaldehyde,
c) introducing the gas phase obtained in step b) into a first condenser and condensing the gas phase in the first condenser to obtain a first condensed phase,
d) separating the first condensed phase obtained in step c) in a first phase separator to obtain an organic phase which comprises methacrolein and an aqueous phase which comprises water, residuals of methacrolein and methanol,
e) introducing the aqueous phase obtained in step d) into a first distillation column, wherein the first distillation column comprises at least one column internal selected from the group consisting of trays and packing materials, and wherein the aqueous phase is introduced into the first distillation column at a first feeding point which lies above the at least one column internal
f) introducing the liquid phase obtained in step b) into the first distillation column wherein the liquid phase is introduced into the first distillation column at a second feeding point which lies below the at least one column internal,
g) distilling the aqueous phase and the liquid phase in the first distillation column to obtain a first overhead stream and a first bottom stream.

2. The process according to claim 1, wherein a side stream is removed from the first distillation column wherein the side stream is removed at a first extraction point, which lies below the first feeding point and above the second feeding point, wherein the side stream comprises mostly water, traces of methacrolein, unconverted formaldehyde and the at least one acid of the homogeneous catalyst mixture.

3. The process according to claim 2, wherein the side stream is at least partially recycled into the first distillation column.

4. The process according to any one of claims 1 to 3, wherein the first overhead stream is obtained at the top of the first distillation column and is introduced into the first condenser.

5. The process according to any one of claims 1 to 4, wherein the first bottom stream is obtained at the bottom of the first distillation column and wherein the first bottom stream has a water content of at least 85 wt.-%, based on the total weight of the first bottom stream.

6. The process according to any one of claims 2 to 5, wherein the side stream is introduced into a second distillation column to obtain a second overhead stream and a second bottom stream, wherein the second overhead stream is at least partially transferred into the first condenser.

7. The process according to any one of claims 1 to 6, wherein a first part of the first bottom stream is recycled into the first reactor and a second part of the first bottom stream is introduced into a third distillation column in which a third overhead stream comprising water and a third bottom stream comprising the homogeneous catalyst mixture is obtained.

8. The process according to claim 7, wherein the third bottom stream obtained in the third distillation column is recycled into the first reactor.

9. The process according to claim 7 or 8, wherein the third bottom stream obtained in the third distillation column comprises in the range from 30 wt.-% to 80 wt.-% water, based on the total weight of the third bottom stream.

10. The process according to any one of claims 7 to 9, wherein the third overhead stream obtained in the third distillation column after condensation has a pH value at 20 °C of more than 7.

11. The process according to any one of claims 1 to 10, wherein the reaction mixture exiting the first reactor has a temperature in the range of from 150 °C to 180 °C.

12. The process according to any one of claims 1 to 11, wherein the aqueous phase obtained in the phase separator has a pH value of less than 7 at 20 °C.

13. The process according to any one of claims 1 to 12, wherein the homogeneous catalyst mixture comprises acetic acid and dimethylamine.

14. The process according to any one of claims 1 to 13, wherein the first distillation column comprises two or more column internals and wherein the second feeding point lies below a first column internal and above a second column internal.

15. The process according to any one of claims 2 to 14, wherein a flow ratio of the aqueous phase of the phase separator into the first distillation column to the liquid phase from the expansion vessel into the first distillation column is in the range from 0.15 to 0.5, preferably in the range from 0.2 to 0.4.
